# EUROPÄISCHE PATENTANMELDUNG

(11) **EP 4 686 939 A1**
(43) Veröffentlichungstag der Anmeldung: **04.02.2026**
(21) Anmeldenummer: 24191532.1
(22) Anmeldetag: 29.07.2024
(51) Int. Cl.: G01N 27/404, G01N 33/00

(54) **ELEKTROCHEMISCHER GASSENSOR UND KORRESPONDIERENDES VERFAHREN**

(71) Anmelder: Testo SE & Co. KGaA, 79822 Titisee-Neustadt (DE)
(72) Erfinder: DORNHOF, Johannes, 79199 Kirchzarten (DE); STEINER, Gregor, 79822 Titisee-Neustad (DE); MÜNCH, Reinhold, 79111 Freiburg (DE)
(74) Vertreter: Mertzlufft-Paufler, Cornelius

(57) **Zusammenfassung**

Elektrochemischer Gassensor (1), mit wenigstens zwei Elektroden (2) und mit einer Spannungsquelle (17), mit der eine Arbeitsspannung zwischen den wenigstens zwei Elektroden (2) anlegbar ist, dadurch gekennzeichnet, dass eine Verstelleinrichtung (20) ausgebildet ist, mit welcher ein Spannungswert der Arbeitsspannung veränderbar ist.

Verfahren zum Betrieb eines elektrochemischen Gassensors (1), wobei zwischen wenigstens zwei Elektroden (2) eine Arbeitsspannung angelegt wird, dadurch gekennzeichnet, dass zu einer Kompensation einer Veränderung einer Potentiallage einer Elektrode (2) der wenigstens zwei Elektroden (2) die Arbeitsspannung verändert wird.

## Beschreibung

Die Erfindung betrifft einen elektrochemischen Sensor mit wenigstens zwei Elektroden und mit einer Spannungsquelle, mit der eine Arbeitsspannung zwischen den wenigstens zwei Elektroden anlegbar ist.

Die Erfindung betrifft weiter ein Verfahren zum Betrieb eines elektrochemischen Gassensors, wobei zwischen wenigstens zwei Elektroden eine Arbeitsspannung angelegt wird.

Elektrochemische Gassensoren sind bekannt und werden beispielsweise dazu eingesetzt, eine Anwesenheit und/oder eine Quantität eines Gases zu detektieren oder nachzuweisen.

Hierzu sind elektrochemische Gassensoren typischerweise mit einem Elektrolyten befüllt, in den mehrere Elektroden eintauchen, wobei in den Elektrolyten an den Elektroden vorbestimmte Redoxreaktionen über eine angelegte Potentialdifferenz stattfinden, die zu einem messbaren Stromfluss führen.

Dabei wird ein Arbeitspunkt des elektrochemischen Gassensors, der sich aus der gewünschten Redoxreaktion, den verwendeten Elektroden und dem Elektrolyten ergibt, durch Einstellung einer Arbeitsspannung festgelegt.

Es hat sich herausgestellt, dass sich der Arbeitspunkt elektrochemischer Gassensoren unbemerkt verlagern kann, z.B. wenn eine zu hohe Konzentration des Analyten oder auch unerwünschte Substanzen in Kontakt mit den Elektroden geraten und so unerwünschte Nebenreaktionen auslösen können.

Die Erfindung befasst sich damit, störende Einflüsse auf den Betrieb eines elektrochemischen Gassensors auf einfache Weise zu vermindern bzw. zu beheben.

Zur Lösung der genannten Aufgabe sind bei einem elektrochemischen Gassensor die Merkmale von Anspruch 1 vorgesehen. Insbesondere wird somit zur Lösung der genannten Aufgabe bei einem elektrochemischen Gassensor der eingangs beschriebenen Art vorgeschlagen, dass eine Verstelleinrichtung ausgebildet ist, mit welcher ein Spannungswert der Arbeitsspannung veränderbar ist. Die Erfindung macht sich die Erkenntnis zunutze, dass eine Verunreinigung eines derartigen elektrochemischen Gassensors durch unerwünschte Substanzen zu einer Potentialverschiebung an wenigstens einer der wenigstens zwei Elektroden führt, die durch eine Änderung der Arbeitsspannung kompensierbar oder zumindest verminderbar ist. Somit kann auf einfache Weise vermieden werden, dass eine Verunreinigung des Gassensors zu unzulässig abweichenden Messergebnissen führt.

Bei einer Ausgestaltung der Erfindung kann vorgesehen sein, dass eine Elektrode der wenigstens zwei Elektroden eine Sensingelektrode ist. Von Vorteil ist dabei, dass eine Änderung der Potentiallage der Referenzelektrode auf einfache Weise kompensierbar ist.

Bei einer Ausgestaltung der Erfindung kann vorgesehen sein, dass eine Elektrode der wenigstens zwei Elektroden eine Referenzelektrode ist. Von Vorteil ist dabei, dass ein Bezugspunkt verwendbar ist, der in der Regel nicht von externen Störgrößen beeinflusst ist.

Bei einer vorteilhaften Ausgestaltung der Erfindung kann vorgesehen sein, dass eine Gegenelektrode und eine Stromstärke-Messeinrichtung ausgebildet sind, wobei mit der Stromstärke-Messeinrichtung eine Stromstärke eines Stromflusses zwischen der Messelektrode und der Gegenelektrode messbar ist. Somit ist auf einfache Weise ein elektrisches Messsignal generierbar, welches mit der an der Sensingelektrode und/oder der Gegenelektrode ablaufenden Redoxreaktion korreliert.

Bei einer vorteilhaften Ausgestaltung kann vorgesehen sein, dass eine Potential-Messeinrichtung ausgebildet ist, mit welcher eine Potentiallage einer Elektrode der wenigstens zwei Elektroden messbar ist. Von Vorteil ist dabei, dass auf einfache Weise eine Veränderung eines Potentials einer Elektrode durchführbar ist. Beispielsweise kann diese Elektrode die bereits erwähnte Referenzelektrode und/oder die bereits erwähnte Gegenelektrode sein.

Bei einer vorteilhaften Ausgestaltung kann vorgesehen sein, dass eine, insbesondere die bereits erwähnte, Potential-Messeinrichtung zur Messung einer Spannung zwischen einer oder der Referenzelektrode und einer oder der Gegenelektrode eingerichtet ist. Somit ist ein einfaches Mittel zur Detektion einer Änderung einer Potentiallage einer Elektrode bereitgestellt.

Bei einer Ausgestaltung der Erfindung kann vorgesehen sein, dass eine Steuereinrichtung ausgebildet ist, mit welcher ein Spannungswert der Arbeitsspannung in Abhängigkeit von einer, insbesondere der bereits erwähnten, Potentiallage einer Elektrode vorgebbar ist. Von Vorteil ist dabei, dass eine Potentialdrift automatisch zumindest teilweise kompensierbar ist. Bevorzugt ist diese Elektrode die Referenzelektrode.

Bei einer Ausgestaltung der Erfindung kann vorgesehen sein, dass die Steuereinrichtung eine Konstantstromquelle hat. Somit ist auf einfache Weise die Arbeitsspannung definiert bereitstellbar.

Bei einer vorteilhaften Ausgestaltung kann vorgesehen sein, dass die Steuereinrichtung einen einstellbaren Widerstand hat. Somit ist auf einfache Weise eine Verstellbarkeit der Arbeitsspannung erreichbar.

Bei einer vorteilhaften Ausgestaltung kann vorgesehen sein, dass die Arbeitsspannung durch einen Spannungsabfall über einem, beispielsweise den erwähnten, einstellbaren Widerstand bestimmt ist.

Alternativ kann die Arbeitsspannung auf andere Weise, beispielsweise auch durch eine variable Stromquelle, beispielsweise einen Operationsverstärker, gebildet sein.

Zur Lösung der genannten Aufgabe sind bei einem Verfahren die Merkmale des nebengeordneten, auf ein Verfahren gerichteten Anspruchs vorgesehen. Insbesondere wird somit bei einem Verfahren der eingangs beschriebenen Art erfindungsgemäß zur Lösung der Aufgabe vorgeschlagen, das zu einer Kompensation einer Veränderung wenigstens einer Potentiallage wenigstens einer Elektrode der wenigstens zwei Elektroden die Arbeitsspannung verändert wird. Somit ist ein Verfahren beschrieben, welches sich leicht automatisieren lässt, beispielsweise dadurch, dass die Veränderung der Arbeitsspannung automatisch aus der Veränderung der Potentiallage bestimmt wird. Besonders vorteilhaft ist die Elektrode, an der die Potentiallage verändert wird, die Referenzelektrode. Es ist aber auch die Sensingelektrode hierzu verwendbar oder beide.

Bei einer Ausgestaltung der Erfindung kann vorgesehen sein, dass die Arbeitsspannung verändert wird, wenn die Veränderung der Potentiallage einen vorbestimmten Schwellenwert übersteigt. Von Vorteil ist dabei, dass eine Anzahl von Änderungen der Arbeitsspannung auf ein geringes Maß begrenzbar ist. Beispielsweise kann der Schwellwert so gewählt sein, dass bei einer Potentialdrift über den Schwellwert hinaus eine bleibende Schädigung des elektrochemischen Gassensors zu befürchten ist oder eine Messcharakteristik des elektrochemischen Gassensors unzulässig deformiert wird.

Bei einer Ausgestaltung der Erfindung kann vorgesehen sein, dass die Veränderung der Potentiallage aus einer Spannungsdifferenz der einen Elektrode, welche bevorzugterweise die Referenzelektrode ist, zu einer Gegenelektrode ermittelt wird. Somit ist eine einfache Möglichkeit zur Bestimmung der Potentialdrift bereitstellbar.

Bei Ausgestaltung der Erfindung kann vorgesehen sein, dass die Arbeitsspannung so eingestellt wird, dass eine rechnerische Spannungsdifferenz zwischen einer, beispielsweise der bereits erwähnten, Sensingelektrode und einer, beispielsweise der bereits erwähnten, Gegenelektrode innerhalb eines (vorgebbaren oder vorgegebenen) Spannungswertfensters liegt. Somit ist ein automatisierbares Kriterium vorgegeben, nach welchem eine Korrektur der Arbeitsspannung vornehmbar ist. Besonders günstig ist es hierbei, wenn die Arbeitsplanung so eingestellt wird, dass die rechnerische Spannungsdifferenz gleich einem Vorgabewert ist.

Eine rechnerische Spannungsdifferenz kann beispielsweise dadurch charakterisierbar sein, dass sie sich aus einer vorzeichenrichtigen Addition gemessener Spannungen ergibt.

Die Erfindung wird nun anhand eines Ausführungsbeispiels näher beschrieben, ist jedoch nicht auf dieses Ausführungsbeispiel beschränkt.

Es zeigt:
- Fig. 1: eine Explosionsdarstellung eines elektrochemischen Gassensors,
- Fig. 2: ein Prinzipschaltbild eines elektrochemischen Gassensors,
- Fig. 3: eine schematische Darstellung von Potentiallage mit zugehörigen Spannungsdifferenzen und
- Fig. 4: eine mögliche Realisierung einer variablen Spannungsquelle der Schaltung gemäß Fig. 2.

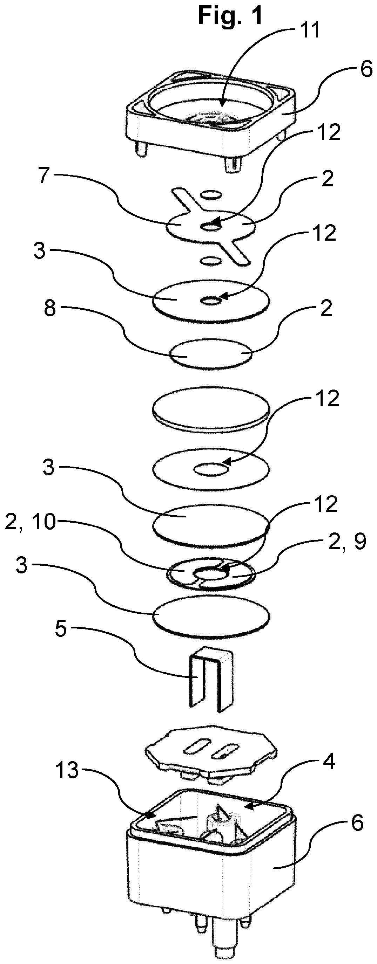

Bei dem in Figur 1 gezeigten Beispiel eines erfindungsgemäßen elektrochemischen Gassensors 1 sind vier Elektroden 2 vorgesehen, zwischen denen elektrolytgetränkte Membranen 3 angeordnet sind. Dieser Elektrolyt wird in einem Vorrat 4 über einen Docht 5 den Membranen 3 zugeführt.

Der Vorrat 4 ist hierbei in dem Gehäuse 6 angeordnet, der auch die Elektroden 2 und Membranen 3 aufnimmt.

Die Elektroden 2 sind hierbei als Sensingelektrode 7, Hilfselektrode 8, Gegenelektrode 9 und Referenzelektrode 10 ausgebildet.

Über einen Gaspfad 11, der durch Öffnungen 12 angedeutet ist, wird den Elektroden 2, die mit dem Elektrolyten, der durch die Membranen 3 bereitgestellt ist, in Kontakt sind, ein Messgas von außen zugeführt. Dieses führt zu einer Redoxreaktion, von denen eine Reaktionsrichtung an der Sensingelektrode 7 und die gegenläufige Reaktion an der Gegenelektrode 9 abläuft.

Zur Einstellung eines Arbeitspunktes wird zwischen die Sensingelektrode 7 und die Referenzelektrode 10 eine Arbeitsspannung angelegt.

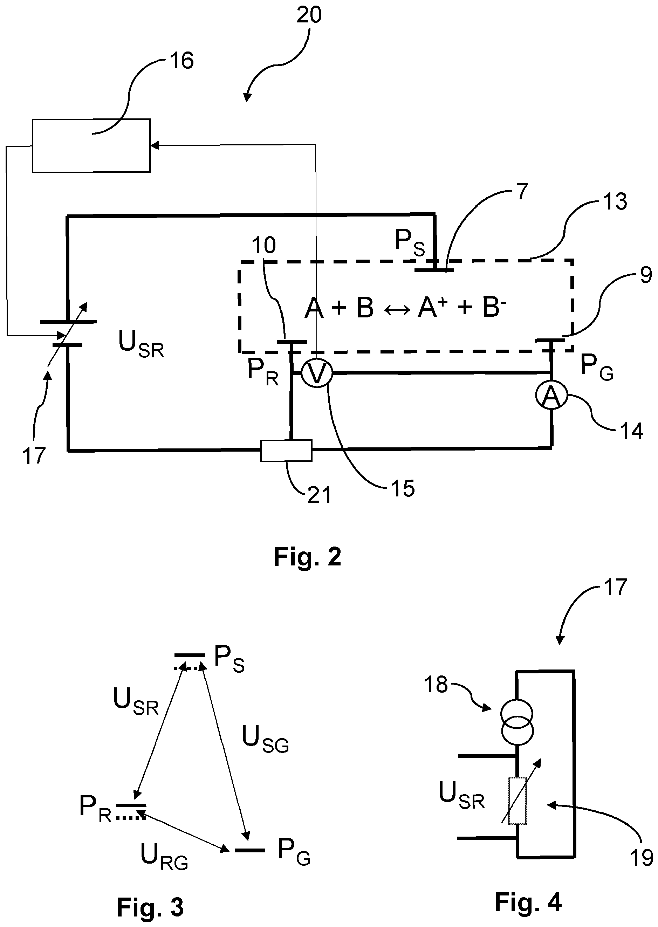

Figur 2 zeigt eine Prinzipdarstellung des elektrochemischen Gassensors 1.

In dem Elektrolyten 13 läuft die Redoxreaktion

A + B ↔ A⁺ + B⁻

ab.

Alle Elektroden tauchen in den Elektrolyten 13 ein.

Aufgrund der Redoxreaktion fließt ein Strom zwischen der Sensingelektrode 7 und der Gegenelektrode 9, der mit einer Stromstärke-Messeinrichtung 14 erfassbar ist.

Damit die gewünschte Reaktion abläuft, wird eine Arbeitsspannung U_{SR} zwischen der Sensingelektrode 7 und der Referenzelektrode 10 angelegt.

Figur 3 zeigt die schematische Darstellung der Potentiallagen P_{S}, P_{G}, P_{R} an der Sensingelektrode (S), Gegenelektrode (G) und Referenzelektrode (R).

Wird durch eine Verunreinigung eine Verschiebung des Potentials P_{R} an der Referenzelektrode 10 bewirkt, so wird die Arbeitsspannung U_{SR} nachgeführt, damit die Potentialdifferenz U_{SG} zwischen der Sensingelektrode 7 und der Gegenelektrode 9 auf einem gewünschten Wert bleibt.

Um eine Drift der Potentiale zu detektieren, kann beispielsweise vorgesehen sein, eine Spannungsdifferenz zwischen dem Potential P_{G} der Gegenelektrode 9 und dem Potential P_{R} der Referenzelektrode 10 zu messen.

Aus dem Diagramm ergibt sich unmittelbar, dass die Potentialdifferenz oder rechnerische Spannungsdifferenz U_{SG} = U_{SR} + U_{RG} ist.

Wird daher diese Spannungsdifferenz U_{RG} mit einer Potential-Messeinrichtung 15 gemessen, so ist es möglich, die Arbeitsspannung U_{SR} zur Kompensation einzustellen.

Figur 2 zeigt ein vereinfachtes Prinzipschaltbild zur Veranschaulichung der elektrochemischen Verhältnisse. Die tatsächliche Beschaltung weicht hiervon in bekannter Weise ab.

Aus Figur 2 ist ersichtlich, dass die Potential-Messeinrichtung 15 zur Messung einer Spannung U_{RG} zwischen der Referenzelektrode R und der Gegenelektrode G eingerichtet ist.

Ein Adierer 21 vermittelt die Potentiale P_{R} und der Spannungsquelle U_{SR} an die Gegenelektrode 9.

Das Signal der Potential-Messeinrichtung 15 steuert eine Steuereinrichtung 16 an. Diese Steuereinrichtung 16 generiert in Abhängigkeit des Eingangssignals ein Steuersignal für die einstellbare Spannungsquelle 17.

Diese Ansteuerung geschieht derart, dass eine Potentialdifferenz U_{SG} zwischen der Sensingelektrode 7 und der Gegenelektrode 9 konstant bleibt (z.B. -1150mV) oder zumindest innerhalb eines vorgegebenen Spannungsfensters liegt.

In Figur 4 ist ersichtlich, dass die variable Spannungsquelle aus einer Standstromquelle 18 und einem einstellbaren Widerstand 19 gebildet ist, wobei die Arbeitsspannung U_{SR} über dem Widerstand 19 abgegriffen wird.

Das Steuersignal der Steuereinrichtung 16 stellt den Widerstandswert des Widerstands 19 ein.

Die Potential-Messeinrichtung 15, die Steuereinrichtung 16 und die variable Spannungsquelle 17 bilden ein Beispiel für eine Verstelleinrichtung 20 für die Arbeitsspannung.

Figur 3 erläutert das Prinzip des erfindungsgemäßen Verfahrens.

Zwischen die Sensingelektrode 7 und die Referenzelektrode 10 wird eine Arbeitsspannung U_{SR} angelegt.

Wenn sich die Potentiallage der Referenzelektrode 10 verändert, was durch eine gestrichene Linie dargestellt ist, würde sich die Potentiallage der Sensingelektrode 10 mitbewegen. Diese Veränderung wird detektiert, und die Arbeitsspannung U_{SR} wird um den gleichen Betrag der Potentialverschiebung jedoch mit inversem Vorzeichen verändert, dass die rechnerische Spannungsdifferenz U_{SG} unverändert bleibt. Bei einer Variante des Verfahrens kann diese Korrektur erst ausgelöst werden, wenn die Potentialdrift einen vorbestimmten Schwellwert übersteigt.

Bei einem elektrochemischen Gassensor 1 wird somit vorgeschlagen, eine Arbeitsspannung, die an wenigstens zwei Elektroden 2 anliegt, so zu verändern, dass eine Potentialdrift an wenigstens einer Elektrode 2 des Gassensors 1 kompensiert wird.

### Bezugszeichenliste

- 1: elektrochemischer Gassensor
- 2: Elektrode
- 3: Membran
- 4: Vorrat
- 5: Docht
- 6: Gehäuse
- 7: Sensingelektrode
- 8: Hilfselektrode
- 9: Gegenelektrode
- 10: Referenzelektrode
- 11: Gaspfad
- 12: Öffnung
- 13: Elektrolyt
- 14: Stromstärken-Messeinrichtung
- 15: Potential-Messeinrichtung
- 16: Steuereinrichtung
- 17: Spannungsquelle
- 18: Konstantstromquelle
- 19: Widerstand
- 20: Verstelleinrichtung
- 21: Adierer

## Patentansprüche

1. Elektrochemischer Gassensor (1), mit wenigstens zwei Elektroden (2) und mit einer Spannungsquelle (17), mit der eine Arbeitsspannung zwischen den wenigstens zwei Elektroden (2) anlegbar ist, **dadurch gekennzeichnet, dass** eine Verstelleinrichtung (20) ausgebildet ist, mit welcher ein Spannungswert der Arbeitsspannung veränderbar ist.

2. Elektrochemischer Gassensor (1) nach Anspruch 1, **dadurch gekennzeichnet, dass** eine Elektrode (2) der wenigstens zwei Elektroden (2) eine Sensingelektrode (7) ist.

3. Elektrochemischer Gassensor (1) nach einem der vorangehenden Ansprüche, **dadurch gekennzeichnet, dass** eine Elektrode (2) der wenigstens zwei Elektroden (2) eine Referenzelektrode (10) ist.

4. Elektrochemischer Gassensor (1) nach einem der vorangehenden Ansprüche, **dadurch gekennzeichnet, dass** eine Gegenelektrode (9) und eine Stromstärke-Messeinrichtung (14) ausgebildet sind, wobei mit der Stromstärke-Messeinrichtung (14) eine Stromstärke eines Stromflusses zwischen der Sensingelektrode (7) und der Gegenelektrode (9) messbar ist.

5. Elektrochemischer Gassensor (1) nach einem der vorangehenden Ansprüche, **dadurch gekennzeichnet, dass** eine Potential-Messeinrichtung (15) ausgebildet ist, mit welcher eine Potentiallage einer Elektrode (2) der wenigstens zwei Elektroden (2), insbesondere der Referenzelektrode (10) und/oder der Gegenelektrode (9), messbar ist.

6. Elektrochemischer Gassensor 1 nach einem der vorangehenden Ansprüche, **dadurch gekennzeichnet, dass** eine oder die Potential-Messeinrichtung (15) zur Messung einer Spannung zwischen einer oder der Referenzelektrode (10) und einer oder der Gegenelektrode (9) eingerichtet ist.

7. Elektrochemischer Gassensor 1 nach einem der vorangehenden Ansprüche, **dadurch gekennzeichnet, dass** eine Steuereinrichtung (16) ausgebildet ist, mit welcher ein Spannungswert der Arbeitsspannung in Abhängigkeit von der Potentiallage einer Elektrode (2), insbesondere einer oder der Referenzelektrode (10) und/oder einer oder der Gegenelektrode (9), vorgebbar ist.

8. Elektrochemischer Gassensor 1 nach einem der vorangehenden Ansprüche, **dadurch gekennzeichnet, dass** die Steuereinrichtung (16) eine Konstantstromquelle (18) hat.

9. Elektrochemischer Gassensor 1 nach einem der vorangehenden Ansprüche, **dadurch gekennzeichnet, dass** die Steuereinrichtung (16) einen einstellbaren Widerstand (19) hat.

10. Elektrochemischer Gassensor 1 nach einem der vorangehenden Ansprüche, **dadurch gekennzeichnet, dass** die Arbeitsspannung durch einen Spannungsabfall über dem oder einem einstellbaren Widerstand (19) bestimmt ist.

11. Verfahren zum Betrieb eines elektrochemischen Gassensors (1), wobei zwischen wenigstens zwei Elektroden (2) eine Arbeitsspannung angelegt wird, **dadurch gekennzeichnet, dass** zu einer Kompensation einer Veränderung einer Potentiallage einer Elektrode (2) der wenigstens zwei Elektroden (2) die Arbeitsspannung verändert wird.

12. Verfahren nach Anspruch 11, **dadurch gekennzeichnet, dass** die Arbeitsspannung (U_{SR}) verändert wird, wenn die Veränderung der Potentiallage einen vorbestimmten Schwellwert übersteigt.

13. Verfahren nach einem der vorangehenden Ansprüche 11 oder 12, **dadurch gekennzeichnet, dass** die Veränderung der Potentiallage aus einer Spannungsdifferenz der einen Elektrode (2) zu einer Gegenelektrode (9) ermittelt wird.

14. Verfahren nach einem der vorangehenden Ansprüche 11 bis 13, **dadurch gekennzeichnet, dass** die Arbeitsspannung so eingestellt wird, dass eine rechnerische Spannungsdifferenz zwischen der oder einer Sensingelektrode (7) und der oder einer Gegenelektrode (9) innerhalb eines Spannungswertfensters liegt, insbesondere gleich einem Vorgabewert ist.

## Geänderte Patentansprüche

### Geänderte Patentansprüche gemäss Regel 137(2) EPÜ.

1. Elektrochemischer Gassensor (1), mit einer Sensingelektrode (7), einer Gegenelektrode (9) und einer Referenzelektrode (10) und mit einer Spannungsquelle (17), mit der eine Arbeitsspannung (U_{SR}) zwischen der Sensingelektrode (7) und der Referenzelektrode (10) anlegbar ist, **dadurch gekennzeichnet, dass** eine Verstelleinrichtung (20) ausgebildet ist, mit welcher ein Spannungswert der Arbeitsspannung (U_{SR}) veränderbar ist, dass eine Potential-Messeinrichtung (15) ausgebildet ist mit welcher eine Spannung (U_{RG}) zwischen der Referenzelektrode (10) und der Gegenelektrode (9) messbar ist und dass eine Steuereinrichtung (16) ausgebildet ist, mit welcher ein Spannungswert der Arbeitsspannung (U_{SR}) in Abhängigkeit von der Spannung (U_{RG}) vorgebbar ist.

2. Elektrochemischer Gassensor (1) nach einem der vorangehenden Ansprüche, **dadurch gekennzeichnet, dass** eine Gegenelektrode (9) und eine Stromstärke-Messeinrichtung (14) ausgebildet sind, wobei mit der Stromstärke-Messeinrichtung (14) eine Stromstärke eines Stromflusses zwischen der Sensingelektrode (7) und der Gegenelektrode (9) messbar ist.

3. Elektrochemischer Gassensor 1 nach einem der vorangehenden Ansprüche, **dadurch gekennzeichnet, dass** die Steuereinrichtung (16) eine Konstantstromquelle (18) hat.

4. Elektrochemischer Gassensor 1 nach einem der vorangehenden Ansprüche, **dadurch gekennzeichnet, dass** die Steuereinrichtung (16) einen einstellbaren Widerstand (19) hat.

5. Elektrochemischer Gassensor 1 nach einem der vorangehenden Ansprüche, **dadurch gekennzeichnet, dass** die Arbeitsspannung durch einen Spannungsabfall über dem oder einem einstellbaren Widerstand (19) bestimmt ist.

6. Verfahren zum Betrieb eines elektrochemischen Gassensors (1), wobei der Gassensor wenigsten eine Sensingelektrode (7), eine Gegenelektrode (9) und eine Referenzelektrode (10) aufweist, wobei zwischen der Sensingelektrode und der Referenzelektrode (10) eine Arbeitsspannung (U_{SR}) angelegt wird, **dadurch gekennzeichnet, dass** zu einer Kompensation einer Veränderung einer Potentiallage der Referenzelektrode (10) die Arbeitsspannung (U_{SR}) verändert wird, wobei die Veränderung der Potentiallage aus einer Spannungsdifferenz (U_{RG}) der Referenzelektrode (10) zu der Gegenelektrode (9) ermittelt wird und dass die Arbeitsspannung (U_{SR}) in Abhängigkeit der ermittelten Spannungsdifferenz (U_{RG}) so eingestellt wird, dass eine rechnerische Spannungsdifferenz (U_{SG}) zwischen der Sensingelektrode (7) und der Gegenelektrode (9) innerhalb eines Spannungswertfensters liegt.

7. Verfahren nach Anspruch 6, **dadurch gekennzeichnet, dass** die Arbeitsspannung (U_{SR}) verändert wird, wenn die Veränderung der Potentiallage einen vorbestimmten Schwellwert übersteigt.
